# EUROPEAN PATENT APPLICATION

(11) **EP 4 850 713 A1**
(43) Date of publication of application: **09.12.2026**
(21) Application number: 25760985.9
(22) Date of filing: 25.02.2025
(51) Int. Cl.: A61M 1/02

(54) **MEDICAL FILTER**

(30) Priority: 29.02.2024 JP 2024029754
(71) Applicant: Terumo Kabushiki Kaisha, Tokyo 151-0072 (JP)
(72) Inventor: KATAOKA, Ryoji, Fujinomiya-shi, Shizuoka 418-0004 (JP)
(74) Representative: TBK
(86) International application number: PCT/JP2025/006369
(87) International publication number: WO 2025/182904

(57) **Abstract**

A medical filter (10) includes a housing (12), an inflow port (14), a filter medium (18), an outflow port (16), and a reinforcing material (19). The reinforcing material (19) allows passage of the liquid (49) that has passed through the filter medium (18) and suppresses deformation of the filter medium (18) due to a pressure of the liquid (49) that has passed through the filter medium (18). The reinforcing material (19) covers the filter medium (18) in an outflow region (42) of the internal space (30) of the housing (12). The reinforcing material (19) has a parallel portion (51) and a tapered portion (53) connected to the parallel portion (51) in a state where the liquid (49) passes therethrough.

## Description

### Technical Field

The present disclosure relates to a medical filter.

### Background Art

JP H07-67952 A discloses a medical filter. The medical filter includes a housing, an inflow port, an outflow port, and a filter medium. The housing has an internal space. The inflow port and the outflow port are each fixed to a peripheral edge portion of the housing. The filter medium is fixed to the housing and disposed in the internal space of the housing. The filter medium separates the internal space of the housing into an inflow region and an outflow region. Liquid (blood) flows into the inflow region through the inflow port. The filter medium blocks passage of a specific component (white blood cells) of the liquid flowing into the inflow region. The liquid having passed through the filter medium flows into the outflow region. The liquid flowing into the outflow region flows out of the housing through the outflow port.

### Citation List

### Patent Literature

Patent Literature 1: JP H07-67952 A

### Summary of Invention

### Technical Problem

In a case where a filter medium is made of a soft material such as polyurethane, the filter medium is deformed by a pressure of liquid flowing into an inflow region and passing through the filter medium. If the filter medium is deformed, a pore size of the filter medium increases, and there is a possibility that a specific component passes through the filter medium. As a result, filtration performance of the filter medium may be deteriorated.

An object of the present invention is to solve the above-described problem.

(1) An aspect of the present invention is a medical filter including: a housing having an internal space; an inflow port that is fixed to a peripheral edge portion of the housing and allows liquid to flow into the internal space from an outside of the housing; a filter medium that is fixed to the housing and is disposed in the internal space; an outflow port that is fixed to the peripheral edge portion and allows the liquid having passed through the filter medium to flow out to the outside; and a reinforcing material that allows the liquid having passed through the filter medium to pass through and suppresses deformation of the filter medium due to a pressure of the liquid passing through the filter medium, in which the filter medium separates the internal space into an inflow region into which the liquid flows through the inflow port and an outflow region from which the liquid having passed through the filter medium flows out to the outside through the outflow port, and the reinforcing material covers the filter medium in the outflow region, and includes a parallel portion and a tapered portion connected to the parallel portion in a state in which the liquid passes through.

According to this configuration, it is possible to suppress deformation of the filter medium by the pressure of the liquid flowing into the inflow region and passing through the filter medium. This results in making it possible to suppress deterioration in filtration performance of the filter medium due to deformation of the filter medium.

(2) In the medical filter according to (1), the reinforcing material may have lower stretchability than the filter medium.

According to this configuration, it is possible to effectively suppress deformation of the filter medium by the pressure of the liquid flowing into the inflow region and passing through the filter medium.

(3) In the medical filter according to (1) or (2), the filter medium may be formed in a bag shape, an inside of the filter medium may be the inflow region, the inflow port may be inserted into the inflow region through a proximal portion of the filter medium, and the reinforcing material may be formed in a bag shape so as to cover an outer surface of the filter medium.

According to this configuration, the filter medium can be easily covered with the reinforcing material.

(4) In the medical filter according to (3), the proximal portion of the filter medium, the inflow port, and a proximal portion of the reinforcing material may be integrally fixed to the peripheral edge portion of the housing.

According to this configuration, the filter medium, the inflow port, and the reinforcing material can be easily fixed inside the housing.

(5) In the medical filter according to (3) or (4), the outer surface of the filter medium and an inner surface of the reinforcing material may be in contact with each other.

According to this configuration, it is possible to effectively suppress deformation of the filter medium by the pressure of the liquid passing through the filter medium.

(6) In the medical filter according to (5), the filter medium and the reinforcing material may be integrally molded.

According to this configuration, the filter medium and the reinforcing material can be easily manufactured.

(7) In the medical filter according to any one of (3) to (6), the parallel portion may be parallel to an axial direction of the inflow port, and the tapered portion may be inclined with respect to the axial direction and connected to the parallel portion so as to surround the parallel portion.

According to this configuration, when the liquid flows into the inflow region, it is possible to effectively suppress the filter medium from swelling in directions orthogonal to the axial direction of the inflow port (a thickness direction and a width direction of the housing) by the pressure of the liquid.

(8) In the medical filter according to any one of (1) to (7), the filter medium may be a porous body that allows passage of the liquid, and the reinforcing material may be a porous body having a pore diameter larger than a pore diameter of the filter medium.

According to this configuration, it is possible to prevent the liquid that has passed through the filter medium from being blocked by the reinforcing material.

(9) In the medical filter according to (8), the reinforcing material may be a nonwoven fabric.

According to this configuration, the filter medium can be easily covered with the reinforcing material in the outflow region.

According to the present invention, it is possible to suppress deformation of the filter medium by the pressure of the liquid flowing into the inflow region and passing through the filter medium. This results in making it possible to suppress deterioration in filtration performance of the filter medium due to deformation of the filter medium.

### Brief Description of Drawings

[Fig. 1] Fig. 1 is a front view of a medical filter according to a first embodiment.
[Fig. 2] Fig. 2 is a cross-sectional view taken along a line II-II of Fig. 1.
[Fig. 3] Fig. 3 is a cross-sectional view taken along a line III-III of Fig. 1.
[Fig. 4] Figs. 4A and 4B are partial cross-sectional views illustrating another configuration example of a filter medium.
[Fig. 5] Fig. 5 is a front view of the medical filter according to the first embodiment in a state where liquid flows into an inflow region.
[Fig. 6] Fig. 6 is a cross-sectional view taken along a line VI-VI of Fig. 5.
[Fig. 7] Fig. 7 is a cross-sectional view of the medical filter.
[Fig. 8] Fig. 8 is a cross-sectional view of the medical filter.
[Fig. 9] Fig. 9 is a cross-sectional view taken along a line IX-IX of Fig. 5.
[Fig. 10] Fig. 10 is a front view of a medical filter according to a comparative example in a state where liquid flows into an inflow region.
[Fig. 11] Fig. 11 is a cross-sectional view taken along a line XI-XI of Fig. 10.
[Fig. 12] Fig. 12 is a cross-sectional view taken along a line XII-XII of Fig. 10.
[Fig. 13] Fig. 13 is a front view of a medical filter according to a second embodiment.
[Fig. 14] Fig. 14 is a front view of a medical filter according to a third embodiment.
[Fig. 15] Fig. 15 is a cross-sectional view taken along a line XV-XV of Fig. 14.
[Fig. 16] Fig. 16 is a front view of a medical filter according to a fourth embodiment.
[Fig. 17] Fig. 17 is a cross-sectional view taken along a line XVII-XVII of Fig. 16.

### Description of Embodiments

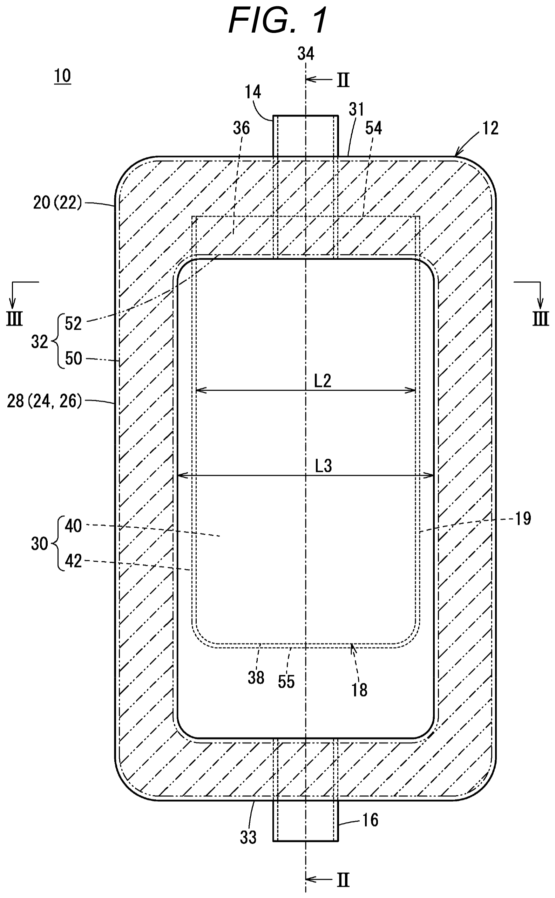

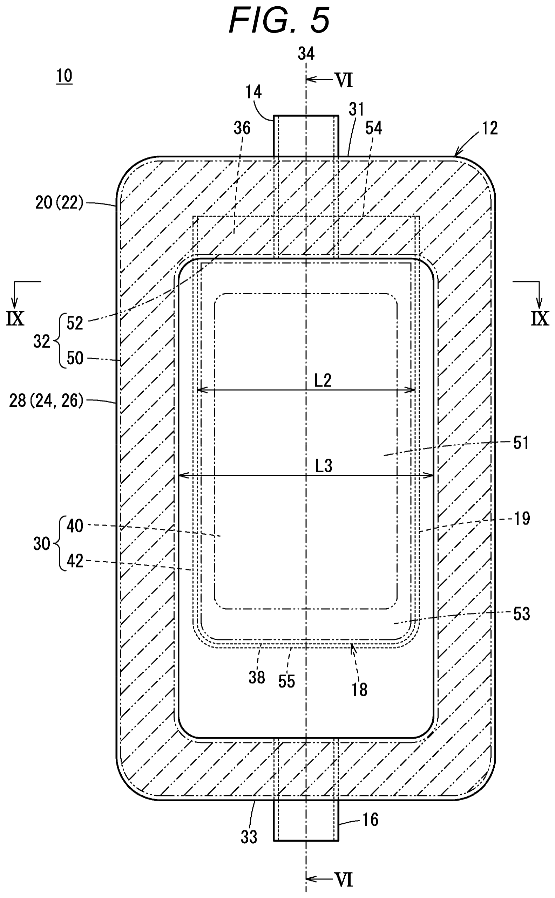

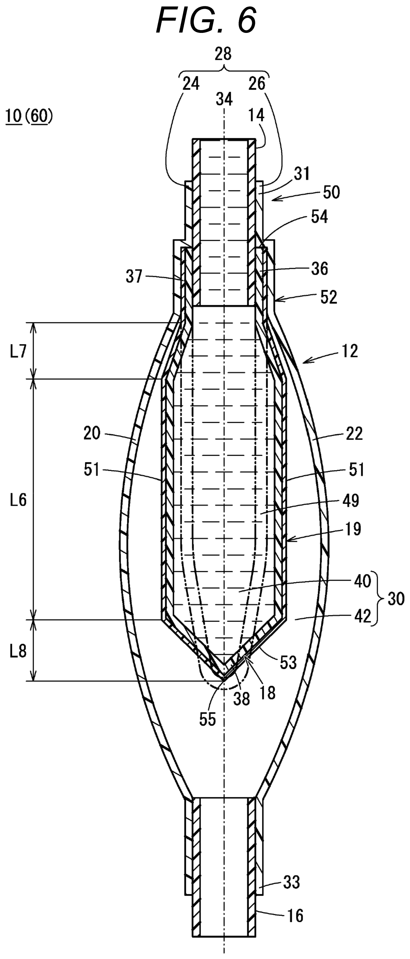

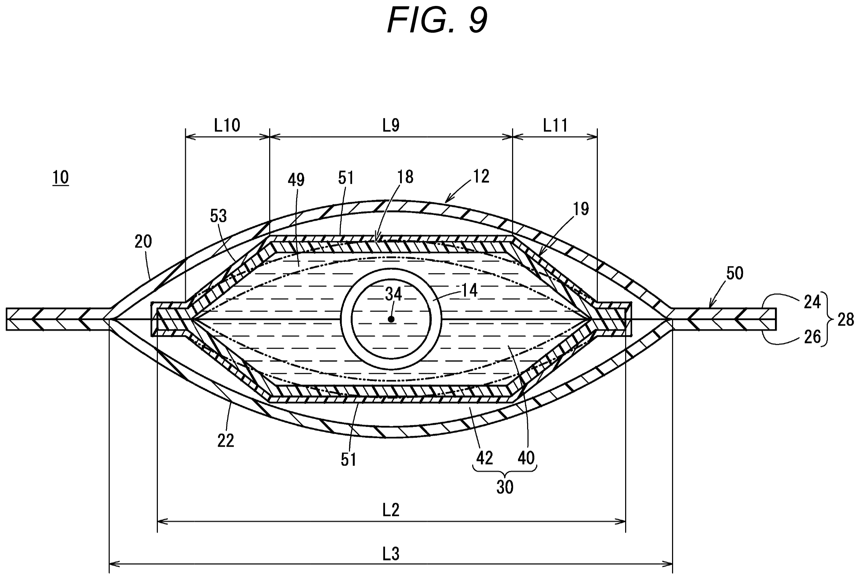

A medical filter 10 according to a first embodiment will be described with reference to Figs. 1 to 9. The medical filter 10 is a filter for filtering liquid 49 (see Figs. 6 and 9). The medical filter 10 is a filter for blocking passage of a predetermined component in the liquid 49. The medical filter 10 can be a filter for blocking passage of a predetermined component (for example, white blood cells) in blood. Note that among Figs. 1 to 9, Figs. 1 to 4B illustrate the medical filter 10 in an initial state in which the liquid 49 is not supplied. Figs. 5 to 9 illustrate the medical filter 10 in a state where the liquid 49 is supplied.

As illustrated in Figs. 1 to 3, the medical filter 10 includes a housing 12, an inflow port 14, an outflow port 16, a filter medium 18, and a reinforcing material 19.

The housing 12 is a rectangular bag body. The housing 12 includes a first sheet portion 20 and a second sheet portion 22. The first sheet portion 20 and the second sheet portion 22 are two separate flexible sheets. The first sheet portion 20 and the second sheet portion 22 are sheets having transparency. The first sheet portion 20 and the second sheet portion 22 are rectangular sheets having the same size and thickness. A constituent material of the first sheet portion 20 and the second sheet portion 22 is synthetic resin. Specific examples of the constituent material of the first sheet portion 20 and the second sheet portion 22 include thermoplastic elastomers such as soft vinyl chloride, polyurethane, an ethylenevinyl acetate copolymer, a hydrogenated product of a styrene-butadiene-styrene copolymer, a styrene-isoprene-styrene copolymer or a hydrogenated product thereof, and mixtures of thermoplastic elastomers and softeners such as polyolefins and ethylene-ethyl acrylate.

The housing 12 is formed in a bag shape by superimposing the first sheet portion 20 and the second sheet portion 22 and fusing an outer edge portion 24 of the first sheet portion 20 and an outer edge portion 26 of the second sheet portion 22. The fusion of the first sheet portion 20 and the second sheet portion 22 may be external heating fusion by heat sealing. Alternatively, the fusion of the first sheet portion 20 and the second sheet portion 22 may be internal fusion by a high-frequency welder, an ultrasonic welder, or the like. In the following description, "fusion" includes the above-described external heating fusion or internal fusion.

The fused outer edge portions 24 and 26 of the first sheet portion 20 and second sheet portion 22 form a peripheral edge portion 28 of the housing 12. Further, by fusing the outer edge portions 24 and 26 of the first sheet portion 20 and the second sheet portion 22, an internal space 30 is formed inside the housing 12. In the following description, the fused portion of the housing 12 is referred to as a fusion region 32. In Fig. 1, the fusion region 32 is illustrated by a two-dot chain line.

Note that the housing 12 may be formed by folding and superimposing one flexible sheet and fusing an outer edge portion of the flexible sheet. In this case, one portion of the folded flexible sheet becomes the first sheet portion 20, and the other portion becomes the second sheet portion 22.

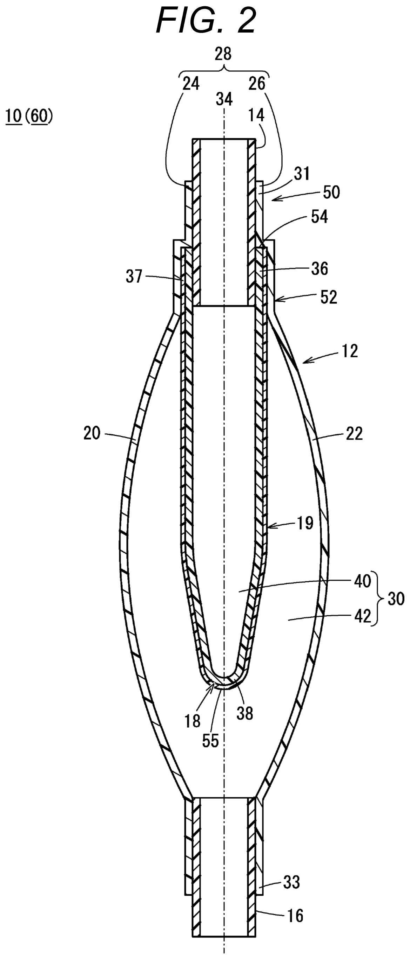

As illustrated in Figs. 1 and 2, the inflow port 14 is a tubular member. The inflow port 14 is provided at a first end portion 31 which is one end portion of the housing 12. Specifically, the inflow port 14 penetrates the first end portion 31 of the peripheral edge portion 28 of the housing 12 and is inserted into the internal space 30 of the housing 12. The inflow port 14 is fixed to the peripheral edge portion 28 of the housing 12 by being fused to the first end portion 31 of the housing 12. A constituent material of the inflow port 14 is, for example, synthetic resin. The inflow port 14 allows the liquid 49 (see Figs. 6 and 9) such as blood to flow into the internal space 30 of the housing 12 from the outside of the housing 12.

The outflow port 16 is a tubular member having the same size and shape as the size and shape of the inflow port 14. The outflow port 16 is provided at a second end portion 33 that is the other end portion of the housing 12. Specifically, the outflow port 16 penetrates the second end portion 33 of the peripheral edge portion 28 of the housing 12 and is inserted into the internal space 30 of the housing 12. The outflow port 16 is fixed to the peripheral edge portion 28 of the housing 12 by being fused to the second end portion 33 of the housing 12. A constituent material of the outflow port 16 is the same as the constituent material of the inflow port 14. The outflow port 16 allows the liquid 49 in the internal space 30 of the housing 12 to flow out of the housing 12.

The inflow port 14 and the outflow port 16 are fixed to the peripheral edge portion 28 of the housing 12 while facing each other. In other words, the inflow port 14 and the outflow port 16 are coaxially fixed to the housing 12. The inflow port 14 and the outflow port 16 are disposed on a central axis 34 of the inflow port 14.

The filter medium 18 is disposed in the internal space 30 of the housing 12. The filter medium 18 is formed in a rectangular shape in a front view of Fig. 1. The filter medium 18 is formed in a bag shape in the cross-sectional view of Fig. 2. The filter medium 18 is fixed to the housing 12 by being fused to the housing 12. As illustrated in Fig. 1, the filter medium 18 is fixed to the housing 12 coaxially with the central axis 34. A proximal portion 36 of the filter medium 18 faces the inflow port 14. A distal portion 38 of the filter medium 18 faces the outflow port 16.

The filter medium 18 is a porous body of synthetic resin or synthetic fiber. Examples of the constituent material of the filter medium 18 include polyester, polyamide, polypropylene, polyethylene, polyurethane, polyvinyl chloride, acrylonitrile, and styrene-based elastomer. A pore size and a thickness of the porous body can be appropriately selected according to a blood component to be separated and removed from blood. For example, in a case where the blood component to be separated and removed from the blood is a white blood cell, the porous body preferably has an average pore size of 3 µm to 20 µm.

The filter medium 18 is formed by folding one rectangular flexible sheet 35 made of the above-described constituent material into a U shape and fusing an outer edge portion of the folded flexible sheet 35. Thus, the filter medium 18 is formed in a bag shape by fusing the outer edge portion of one flexible sheet 35. Alternatively, the filter medium 18 may be formed in a bag shape by superimposing two flexible sheets 35 made of the above-described constituent material and fusing outer edge portions of the two flexible sheets 35.

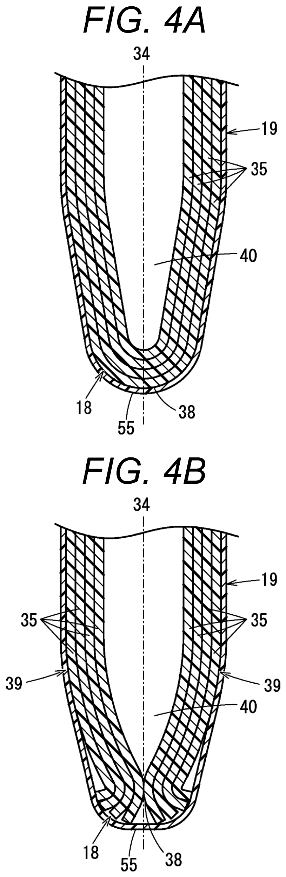

The filter medium 18 may be formed as illustrated in Figs. 4A and 4B.

The filter medium 18 illustrated in Fig. 4A is formed by superimposing a plurality of rectangular flexible sheets 35, folding them into a U shape, and fusing the outer edge portions of the plurality of folded flexible sheets 35.

The filter medium 18 illustrated in Fig. 4B is formed in a bag shape by superimposing a plurality of rectangular flexible sheets 35 and fusing outer edge portions of the plurality of flexible sheets 35. Specifically, one sheet body 39 in which a plurality of flexible sheets 35 is superimposed and the other sheet body 39 in which a plurality of flexible sheets 35 is superimposed are prepared. An outer edge portion of the one sheet body 39 and an outer edge portion of the other sheet body 39 are fused to form the filter medium 18 in a bag shape. In this case, the outer edge portion of the one sheet body 39 is an outer edge portion of the plurality of flexible sheets 35 constituting the one sheet body 39. The outer edge portion of the other sheet body 39 is an outer edge portion of the plurality of flexible sheets 35 constituting the other sheet body 39.

As illustrated in Figs. 1 and 2, the inflow port 14 passes through the proximal portion 36 of the bag-shaped filter medium 18. In other words, the inflow port 14 communicates with the inside of the filter medium 18. The housing 12, the proximal portion 36 of the filter medium 18, and the inflow port 14 are integrally fused. As a result, the proximal portion 36 of the filter medium 18 and the inflow port 14 are integrally fixed to the housing 12.

The filter medium 18 separates the internal space 30 of the housing 12 into an inflow region 40 and an outflow region 42. Specifically, the inflow region 40 is a space inside the filter medium 18. The inflow region 40 communicates with the outside of the housing 12 via the inflow port 14. The liquid 49 (see Figs. 6 and 9) flows into the inflow region 40 through the inflow port 14.

The filter medium 18 blocks passage of a predetermined component (for example, white blood cells in blood) in the liquid 49 flowing into the inflow region 40. The filter medium 18 allows passage of components other than the predetermined component in the liquid 49.

The outflow region 42 is a space outside the filter medium 18 in the internal space 30 of the housing 12. The outflow region 42 communicates with the outflow port 16. The liquid 49 that has passed through the filter medium 18 flows into the outflow region 42. The liquid 49 flowing into the outflow region 42 flows out of the housing 12 via the outflow port 16.

When the liquid 49 flows into the inflow region 40, the filter medium 18 receives the pressure of the liquid 49. The reinforcing material 19 is disposed in the internal space 30 to suppress deformation of the filter medium 18 due to the pressure of the liquid 49. Thus, as will be described later, the reinforcing material 19 is made of a material that is harder than the filter medium 18 and has lower stretchability (tensile elongation) than the filter medium 18.

Specifically, the reinforcing material 19 covers an outer surface of the filter medium 18 in the outflow region 42. The filter medium 18 is formed in a bag shape, and thus, the reinforcing material 19 is formed in a bag shape so as to cover the outer surface of the filter medium 18. Thus, the entire outer surface of the filter medium 18 is covered with the reinforcing material 19. The filter medium 18 and the reinforcing material 19 overlap each other. In other words, the outer surface of the filter medium 18 and an inner surface of the reinforcing material 19 are in contact with each other. In this case, the outer surface of the filter medium 18 and the inner surface of the reinforcing material 19 may be fused. The filter medium 18 and the reinforcing material 19 may be integrally formed. Further, as illustrated in Figs. 4A and 4B, in a case where the filter medium 18 is formed by superimposing a plurality of flexible sheets 35, the reinforcing material 19 is disposed so as to cover the outer surface of the outermost flexible sheet 35.

When the liquid 49 flows into the inflow region 40, the filter medium 18 receives the pressure of the liquid 49 and swells in directions orthogonal to an axial direction of the inflow port 14. In addition, as the filter medium 18 swells in the directions orthogonal to the axial direction of the inflow port 14, the distal portion 38 side of the filter medium 18 (the bottom portion of the filter medium 18) is pulled (pulled up) toward the inflow port 14. The axial direction of the inflow port 14 is a direction along the central axis 34. The directions orthogonal to the axial direction of the inflow port 14 are a width direction and a thickness direction of the housing 12. Note that the width direction of the housing 12 is also a width direction of the filter medium 18 and the reinforcing material 19. The thickness direction of the housing 12 is also a thickness direction of the filter medium 18 and the reinforcing material 19. In Figs. 6 to 9, shapes of the filter medium 18 and the reinforcing material 19 when the liquid 49 flows into the inflow region 40 are illustrated by solid lines, and shapes of the filter medium 18 and the reinforcing material 19 in the initial state in which the liquid 49 does not flow into the inflow region 40 are illustrated by two-dot chain lines.

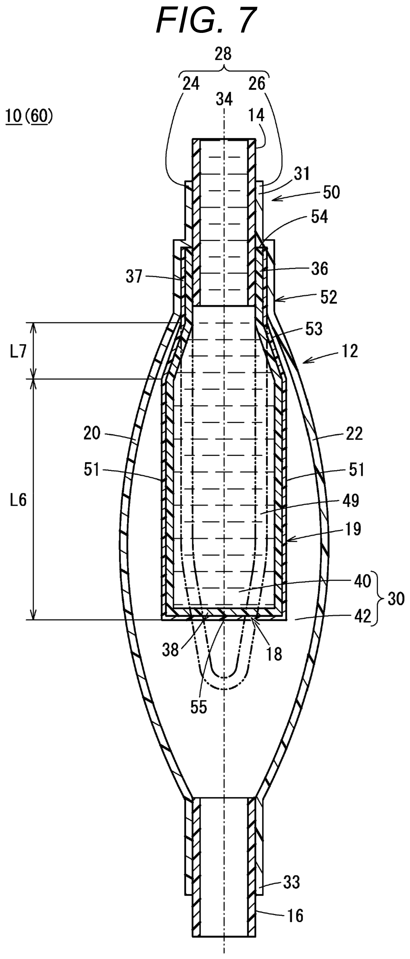

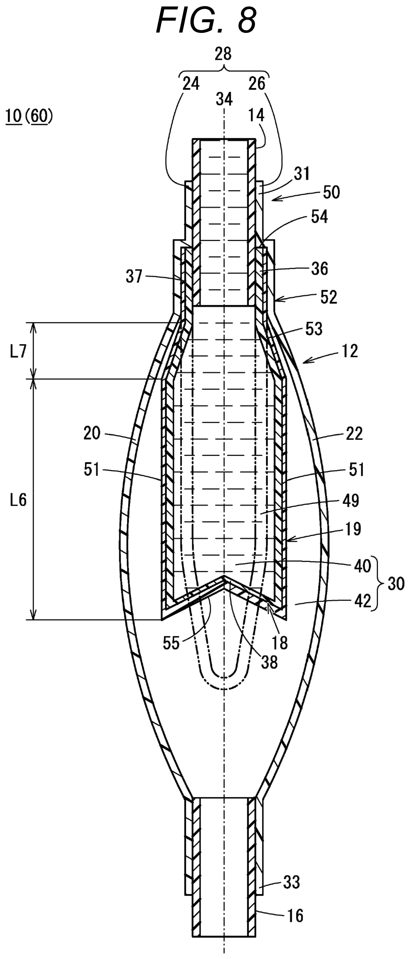

As described above, the reinforcing material 19 covers the outer surface of the filter medium 18. Thus, when the liquid 49 flows into the inflow region 40 and the liquid 49 passes through the filter medium 18, the reinforcing material 19 also receives the pressure of the liquid 49 and swells in the directions orthogonal to the axial direction of the inflow port 14. More specifically, when the liquid 49 flows into the inflow region 40, the pressure of the liquid 49 is uniformly applied to the inside of the filter medium 18. As a result, the filter medium 18 tends to expand in the thickness direction and the width direction of the filter medium 18. As described above, the reinforcing material 19 is provided outside the filter medium 18 and is harder and has lower tensile elongation than the filter medium 18. As a result, the pressure of the liquid 49 is uniformly applied, so that it is possible to suppress expansion of the filter medium 18 in the thickness direction and the width direction. Further, a portion on the distal portion 55 side (bottom portion) of the reinforcing material 19 is a relatively soft portion. Thus, when the liquid 49 flows into the inflow region 40 and the reinforcing material 19 receives the pressure of the liquid 49, the distal portion 55 side of the reinforcing material 19 is pulled up to the inflow port 14 side. Fig. 6 illustrates a state in which the distal portion 55 side (bottom portion) of the reinforcing material 19 and the distal portion 38 side (bottom portion) of the filter medium 18 are slightly pulled up toward the inflow port 14 as compared with a state before the liquid 49 flows into the inflow region 40 (two-dot chain line). Fig. 7 illustrates a state in which the bottom portion of the reinforcing material 19 and the bottom portion of the filter medium 18 are pulled up in a planar shape toward the inflow port 14. Fig. 8 illustrates a state in which the bottom portion of the reinforcing material 19 and the bottom portion of the filter medium 18 are pulled up toward the inflow port 14, and a central portion of each bottom portion is recessed toward the inflow port 14.

When the liquid 49 flows into the inflow region 40, the reinforcing material 19 swells in a hexagonal shape as viewed from the width direction of the housing 12 (see Fig. 6). In addition, the reinforcing material 19 swells in a hexagonal shape as viewed from the axial direction of the inflow port 14 (see Fig. 9).

In other words, in a state where the liquid 49 flows into the inflow region 40, the two parallel portions 51 and the tapered portion 53 are formed in the reinforcing material 19. In other words, in a state where the liquid 49 flows into the inflow region 40, the reinforcing material 19 has the two parallel portions 51 and the tapered portion 53.

Each of the two parallel portions 51 is a portion of the reinforcing material 19 parallel to the central axis 34. Each of the two parallel portions 51 is a flat portion parallel to the central axis 34. The two parallel portions 51 face each other across the central axis 34. Each of the two parallel portions 51 is separated from the central axis 34 in the thickness direction of the housing 12 as compared with the initial state. In other words, each of the two parallel portions 51 protrudes in the thickness direction of the housing 12 as compared with the initial state.

The tapered portion 53 is connected to the two parallel portions 51. The tapered portion 53 is connected to the two parallel portions 51 so as to surround each of the two parallel portions 51. The tapered portion 53 is inclined with respect to the central axis 34. Specifically, a proximal side of the tapered portion 53 connects the proximal portion 37 of the reinforcing material 19 and the two parallel portions 51. Thus, the proximal side of the tapered portion 53 is inclined from the two parallel portions 51 toward the proximal portion 37 of the reinforcing material 19. A distal side of the tapered portion 53 is inclined from the two parallel portions 51 toward the distal portion 55 of the reinforcing material 19. Furthermore, a side portion of the tapered portion 53 between the distal side and the proximal side of the tapered portion 53 is inclined toward a side edge of the reinforcing material 19.

The reinforcing material 19 is not provided with a fold line for forming a boundary between the two parallel portions 51 and the tapered portion 53. The liquid 49 flows into the inflow region 40, and the two parallel portions 51 and the tapered portion 53 are formed by receiving the pressure of the liquid 49 that has passed through the filter medium 18. In Fig. 6, in the axial direction of the inflow port 14, a width L6 of each of the two parallel portions 51 is larger than a width L7 on the proximal side and a width L8 on the distal side of the tapered portion 53 (L6 > L7, L6 > L8). Further, as illustrated in Fig. 9, in the width direction of the housing 12, a width L9 of each of the two parallel portions 51 is larger than widths L10 and L11 of the side portions of the tapered portion 53 (L9 > L10, L9 > L11).

The two parallel portions 51 and the tapered portion 53 are formed in the reinforcing material 19 in a state where the liquid 49 flows into the inflow region 40, and thus, swelling of the filter medium 18 is suppressed inside the reinforcing material 19 even if the filter medium 18 swells by receiving the pressure of the liquid 49. In other words, swelling of the filter medium 18 is suppressed to a shape along the inner surface of the reinforcing material 19. This makes it possible to suppress swelling of the filter medium 18 in the thickness direction and the width direction of the housing 12.

If the inflow of the liquid 49 into the inflow region 40 is stopped, the reinforcing material 19 and the filter medium 18 return to the original shapes (shapes in the initial state).

The reinforcing material 19 is a porous body that allows passage of the liquid 49 that has passed through the filter medium 18. A pore diameter of the porous body constituting the reinforcing material 19 is the same as the pore diameter of the porous body constituting the filter medium 18. Alternatively, the pore diameter of the porous body constituting the reinforcing material 19 is larger than the pore diameter of the porous body constituting the filter medium 18. In this case, the porous body constituting the reinforcing material 19 is coarser than the porous body constituting the filter medium 18. Thus, the reinforcing material 19 does not filter the liquid 49 that has passed through the filter medium 18. In other words, the reinforcing material 19 does not function as a filter for the liquid 49.

The reinforcing material 19 is a porous body that is harder than the filter medium 18 and has lower stretchability than the filter medium 18. Thus, when the filter medium 18 and the reinforcing material 19 receive the same force (for example, the pressure of the liquid 49), the reinforcing material 19 is less likely to stretch than the filter medium 18. In other words, the reinforcing material 19 has higher rigidity against tensile force than the filter medium 18.

Examples of the porous body constituting the reinforcing material 19 include a nonwoven fabric made of PET. In a case where the porous body constituting the reinforcing material 19 is the nonwoven fabric made of PET, a diameter of the fiber constituting the nonwoven fabric is preferably about 0.3 µm to 20 µm. A basis weight per 0.1 mm of the thickness of the nonwoven fabric is preferably about 10 g/m² to 60 g/m². The basis weight is more preferably about 20 g/m² to 50 g/m².

Further, a surface of the porous body (for example, a nonwoven fabric) constituting the reinforcing material 19 may be coated with a hydrophilic polymer or an antithrombotic material. This provides favorable passability of platelets through the nonwoven fabric.

In addition, a pre-filter portion may be provided inside the filter medium 18. The pre-filter portion separates and removes agglomerates, and the like, from blood. In a case where the pre-filter portion is a nonwoven fabric, a diameter of the fiber constituting the nonwoven fabric is preferably about 5 µm to 30 µm. A basis weight per 0.1 mm of the thickness of the nonwoven fabric is preferably about 10 g/m² to 40 g/m². The basis weight is more preferably about 15 g/m² to 35 g/m².

Alternatively, the reinforcing material 19 may be made of a mesh-like member having lower stretchability than the filter medium 18 and a larger hole diameter than the filter medium 18.

The reinforcing material 19 may be made of a porous body having lower stretchability (tensile elongation) than the filter medium 18. Thus, the reinforcing material 19 may be made of a porous body made of a material other than PET. Alternatively, the porous body constituting the reinforcing material 19 may be a porous body having lower stretchability than the filter medium 18 and the same material as the filter medium 18.

The tensile elongation of the reinforcing material 19 will be described in detail. In a case of being viewed from the axial direction of the inflow port 14, a ratio of a circumferential length of the reinforcing material 19 in a state in which the liquid 49 flows into the inflow region 40 with reference to a circumferential length of the reinforcing material 19 in the initial state in which the liquid 49 does not flow into the inflow region 40 is referred to as tensile elongation of the reinforcing material 19. In this case, the tensile elongation of the reinforcing material 19 is preferably 0% to 40%. The tensile elongation is more preferably 0% to 35%. The tensile elongation is further more preferably 0% to 30%. The tensile elongation is still further more preferably 0% to 25%. The tensile elongation is yet further more preferably 0% to 20%. By setting the tensile elongation of the reinforcing material 19 in this manner, it is possible to effectively suppress swelling of the filter medium 18 in the width direction and the thickness direction of the housing 12 when the liquid 49 flows into the inflow region 40.

As illustrated in Figs. 1 and 2, the fusion region 32 has a peripheral edge fusion region 50 and a filter medium fusion region 52.

The peripheral edge fusion region 50 is a fused portion between the outer edge portion 24 of the first sheet portion 20 and the outer edge portion 26 of the second sheet portion 22 in the peripheral edge portion 28 of the housing 12. In the peripheral edge fusion region 50, the outer edge portion 24 of the first sheet portion 20 and the outer edge portion 26 of the second sheet portion 22 are directly fused.

The peripheral edge fusion region 50 is integrally fused with the inflow port 14 and the outflow port 16.

An opening of the inflow port 14 on the outflow port 16 side (an opening communicating with the inflow region 40) is open at a position of the filter medium fusion region 52. Alternatively, the opening of the inflow port 14 on the outflow port 16 side may be open at a position closer to the outflow port 16 than the filter medium fusion region 52.

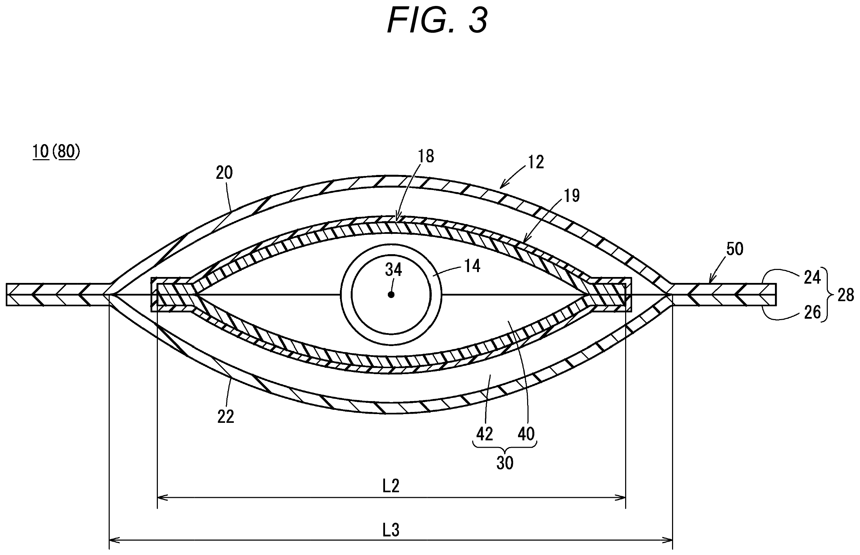

The filter medium fusion region 52 is formed inside the peripheral edge fusion region 50 in the width direction of the housing 12. The filter medium fusion region 52 is a fused portion of the housing 12 with the filter medium 18. The filter medium fusion region 52 extends along the width direction (the left-right direction in Figs. 1 and 3) of the housing 12 orthogonal to the axial direction of the inflow port 14 and the thickness direction of the housing 12.

As illustrated in Fig. 1, the filter medium fusion region 52 is connected to the peripheral edge fusion region 50. Specifically, both ends of the filter medium fusion region 52 in the width direction and the proximal end of the filter medium fusion region 52 in the axial direction are connected to the peripheral edge fusion region 50. The filter medium fusion region 52 is integrally fused to the proximal portion 36 of the filter medium 18, the proximal portion 37 of the reinforcing material 19, and the inflow port 14. Although schematically illustrated in Fig. 2, strictly speaking, in the filter medium fusion region 52, the filter medium 18 and the reinforcing material 19 are impregnated with part of the housing 12 (the first sheet portion 20 and the second sheet portion 22). In the filter medium fusion region 52, an outer peripheral surface of the inflow port 14 and an outer peripheral surface of the reinforcing material 19 are surrounded by the filter medium 18 and the housing 12. Thus, in a portion where the inflow port 14 is located in the filter medium fusion region 52, the filter medium 18 and the reinforcing material 19 are impregnated with part of the housing 12, so that the housing 12, the filter medium 18, the reinforcing material 19, and the inflow port 14 are integrally fused. In the filter medium fusion region 52, the first sheet portion 20, the filter medium 18, the reinforcing material 19, and the second sheet portion 22 are integrally fused by the filter medium 18 and the reinforcing material 19 being impregnated with part of the housing 12 except for a portion where the inflow port 14 is located.

As illustrated in Figs. 1 and 3, the width L3 which is a dimension of the outflow region 42 in the width direction is larger than the width L2 which is a dimension of the filter medium 18 in the width direction (L3 > L2). The outflow region 42 extends toward the outflow port 16 from the distal portion 38 of the filter medium 18.

The medical filter 10 according to the first embodiment is used as follows.

The liquid 49 such as blood is fed by a pump to flow into the inflow region 40 from the outside of the housing 12 via the inflow port 14. The filter medium 18 blocks passage of a specific component such as white blood cells and allows passage of components other than the specific component for the liquid 49 flowing into the inflow region 40. The liquid 49 that has passed through the filter medium 18 passes through the reinforcing material 19 and flows into the outflow region 42. The liquid 49 flowing into the outflow region 42 flows out of the housing 12 via the outflow port 16.

In this event, the filter medium 18 receives the pressure of the liquid 49 and swells in directions orthogonal to the axial direction of the inflow port 14. Further, the bottom portion of the filter medium 18 is pulled up toward the inflow port 14. In addition, the reinforcing material 19 covers the outer surface of the filter medium 18, and thus, the reinforcing material 19 also swells in the directions orthogonal to the axial direction of the inflow port 14 under the pressure of the liquid 49, and the bottom portion of the reinforcing material 19 is pulled up toward the inflow port 14. In this event, the two parallel portions 51 and the tapered portion 53 are formed on the reinforcing material 19. The reinforcing material 19 has lower stretchability (tensile strength) than the filter medium 18. Thus, when the liquid 49 passes through the filter medium 18, it is possible to suppress the filter medium 18 from being deformed by the pressure of the liquid 49. In other words, swelling of the filter medium 18 can be suppressed to the inside of the reinforcing material 19. This makes it possible to suppress the filter medium 18 from swelling in the directions orthogonal to the axial direction of the inflow port 14 by the pressure of the liquid 49.

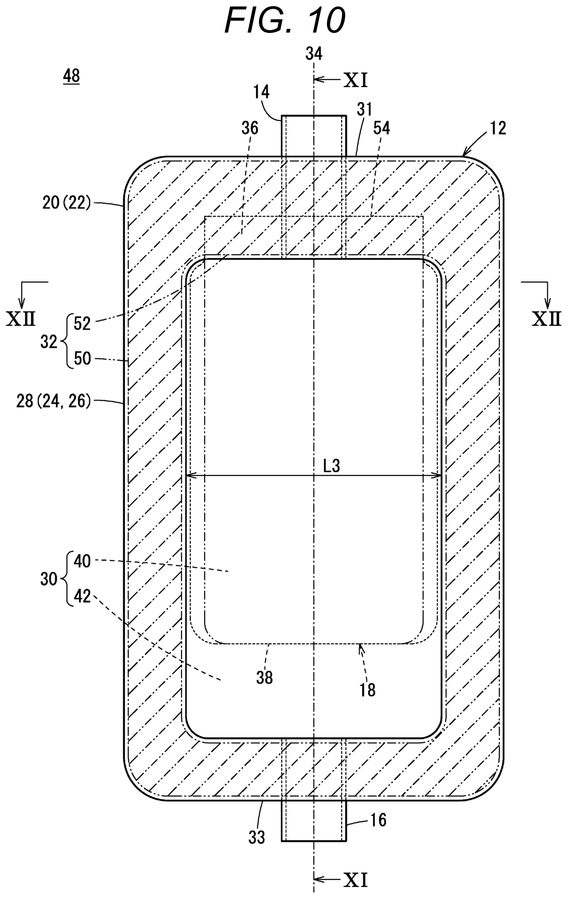

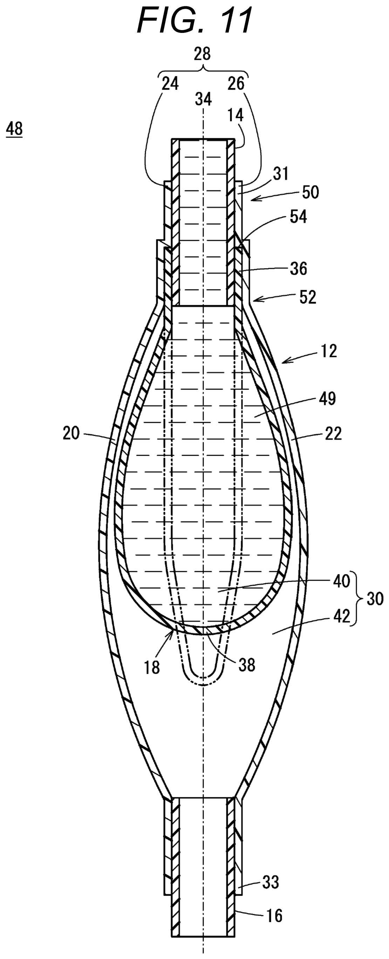

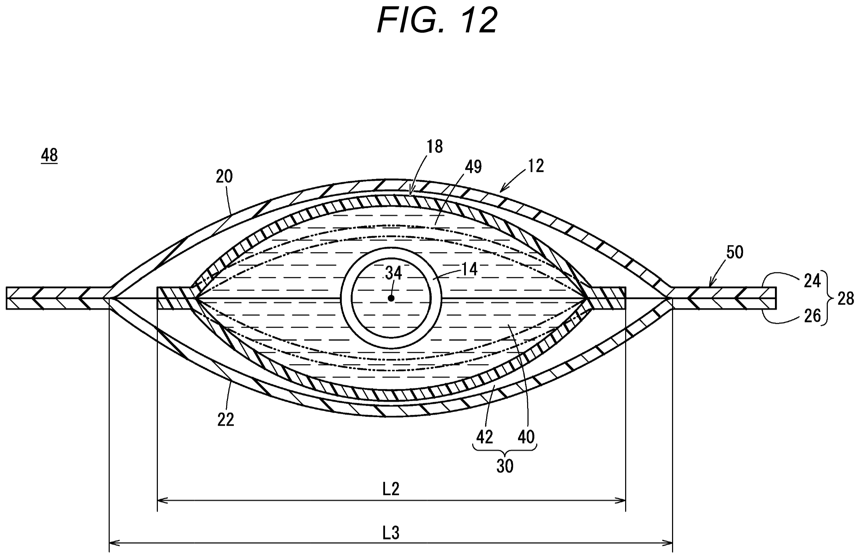

Figs. 10 to 12 illustrate a medical filter 48 according to a comparative example. The medical filter 48 according to the comparative example does not include the reinforcing material 19.

In the medical filter 48, when the liquid 49 flows into the inflow region 40, the filter medium 18 swells by receiving the pressure of the liquid 49. Specifically, when the liquid 49 flows into the inflow region 40, the filter medium 18 receives the pressure of the liquid 49, swells in directions orthogonal to the axial direction of the inflow port 14, and the bottom portion of the filter medium 18 is pulled up toward the inflow port 14. As a result, the pore size of the filter medium 18 increases, and the filtration performance of the filter medium 18 decreases.

On the other hand, in the medical filter 10 according to the first embodiment, the reinforcing material 19 is provided, and thus, swelling of the filter medium 18 is suppressed inside the reinforcing material 19. This suppresses deformation of the filter medium 18, so that the filtration performance of the filter medium 18 can be maintained.

The first embodiment has the following effects.

In a case where the reinforcing material 19 is not provided, when the liquid 49 flowing into the inflow region 40 passes through the filter medium 18, the filter medium 18 may be deformed by the pressure of the liquid 49. Specifically, the filter medium 18 may swell in directions orthogonal to the axial direction of the inflow port 14 (the thickness direction and the width direction of the housing 12) by the pressure of the liquid 49 passing through the filter medium 18. As a result of the filter medium 18 swelling, the pore size of the porous body constituting the filter medium 18 increases, and there is a possibility that the filtration performance of the filter medium 18 may be deteriorated.

On the other hand, in the first embodiment, as illustrated in Figs. 2 and 3, the reinforcing material 19 is disposed in the internal space 30. The reinforcing material 19 allows passage of the liquid 49 that has passed through the filter medium 18 and covers the filter medium 18 in the outflow region 42. In addition, the reinforcing material 19 has the parallel portion 51 and the tapered portion 53 connected to the parallel portion 51 in a state where the liquid 49 passes therethrough. This makes it possible to suppress the filter medium 18 from being deformed by the pressure of the liquid 49 flowing into the inflow region 40 and passing through the filter medium 18. This makes it possible to suppress deterioration in filtration performance of the filter medium 18 due to deformation of the filter medium 18.

The reinforcing material 19 has lower stretchability than the filter medium 18, so that it is possible to effectively suppress the filter medium 18 from being deformed by the pressure of the liquid 49 flowing into the inflow region 40 and passing through the filter medium 18.

The reinforcing material 19 is formed in a bag shape so as to cover the outer surface of the bag-shaped filter medium 18. Thus, the filter medium 18 can be easily covered with the reinforcing material 19.

The proximal portion 36 of the filter medium 18, the inflow port 14, and the proximal portion 37 of the reinforcing material 19 are integrally fixed to the peripheral edge portion 28 of the housing 12. As a result, the filter medium 18, the inflow port 14, and the reinforcing material 19 can be easily fixed inside the housing 12.

The outer surface of the filter medium 18 and the inner surface of the reinforcing material 19 are in contact with each other, so that it is possible to effectively suppress deformation of the filter medium 18 by the pressure of the liquid 49 passing through the filter medium 18.

By integrally molding the filter medium 18 and the reinforcing material 19, the filter medium 18 and the reinforcing material 19 can be easily manufactured.

The parallel portion 51 is parallel to the axial direction of the inflow port 14, and the tapered portion 53 is inclined with respect to the axial direction and is connected to the parallel portion 51 so as to surround the parallel portion 51. As a result, when the liquid 49 flows into the inflow region 40, it is possible to effectively suppress the filter medium 18 from swelling in the directions orthogonal to the axial direction of the inflow port 14 by the pressure of the liquid 49.

The filter medium 18 is a porous body that allows passage of the liquid 49, and the reinforcing material 19 is a porous body having a pore diameter larger than the pore diameter of filter medium 18. This makes it possible to prevent the liquid 49 that has passed through the filter medium 18 from being blocked by the reinforcing material 19.

When the reinforcing material 19 is a nonwoven fabric, the filter medium 18 can be easily covered with the reinforcing material 19 in the outflow region 42.

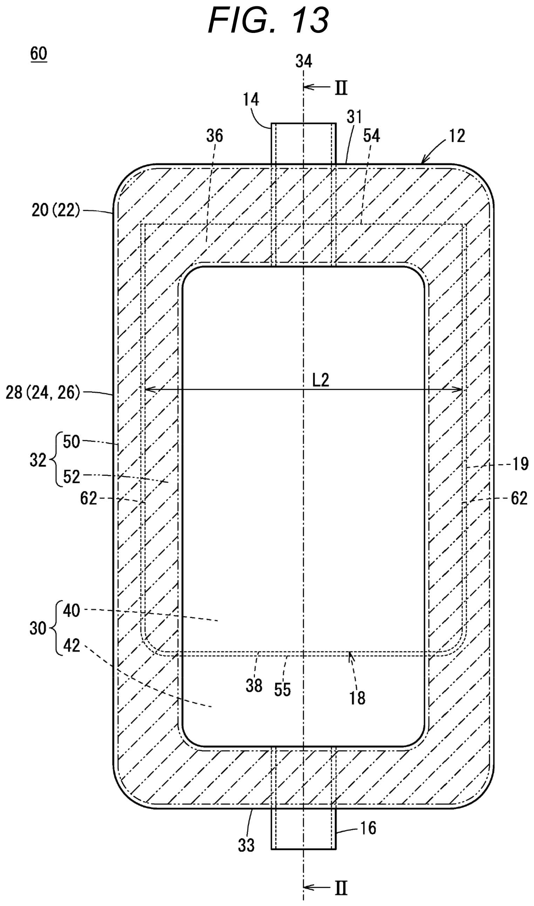

A medical filter 60 according to a second embodiment will be described with reference to Fig. 13. In the medical filter 60 according to the second embodiment, the same components as those of the medical filter 10 according to the first embodiment (see Figs. 1 to 9) are denoted by the same reference numerals, and detailed description thereof will be omitted.

In the medical filter 60 according to the second embodiment, the filter medium fusion region 52 is integrally fused to two side edge portions 62 between the proximal portion 36 and the distal portion 38 of the filter medium 18, the proximal portion 36 of the filter medium 18, and the inflow port 14. Thus, the filter medium fusion region 52 is formed in a U shape. Note that the filter medium fusion region 52 may be integrally fused to at least one side edge portion 62 of the two side edge portions 62 of the filter medium 18, the proximal portion 36 of the filter medium 18, and the inflow port 14.

Also in the second embodiment, effects similar to those of the first embodiment can be obtained.

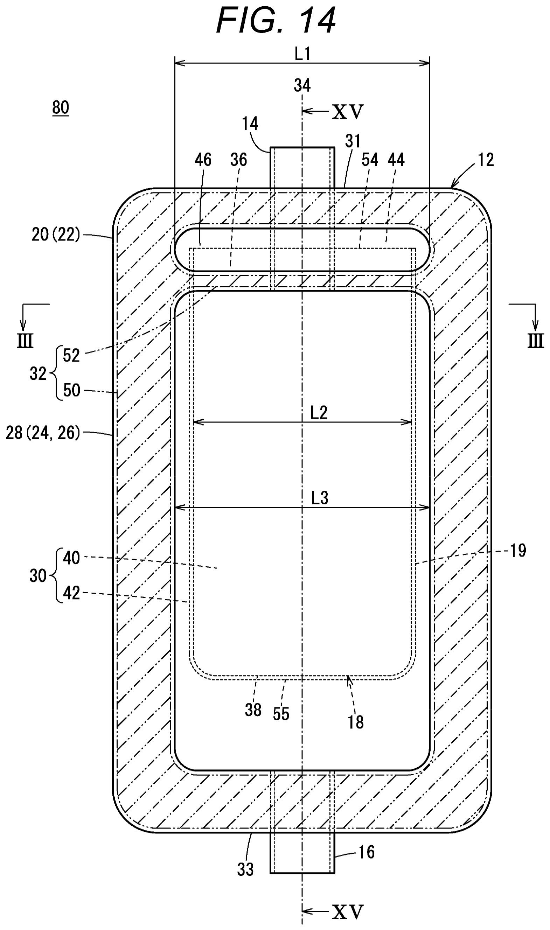

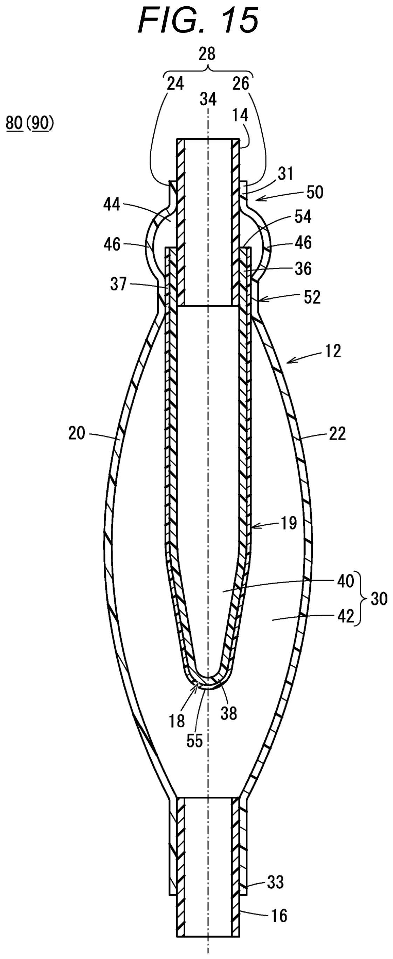

A medical filter 80 according to a third embodiment will be described with reference to Figs. 14 and 15.

In the third embodiment, the housing 12 further includes a non-fusion region 44 and a window portion 46.

The non-fusion region 44 is a space formed inside the housing 12. The non-fusion region 44 is a space different from the internal space 30 of the housing 12. The non-fusion region 44 is a space region that is not fused. The non-fusion region 44 is surrounded by the fusion region 32.

In the third embodiment, only both ends of the filter medium fusion region 52 in the width direction are connected to the peripheral edge fusion region 50. In the third embodiment, the filter medium fusion region 52 separates the inside of the housing 12 into the internal space 30 and the non-fusion region 44. Specifically, the filter medium fusion region 52 separates the non-fusion region 44 and the outflow region 42 of the internal space 30.

The non-fusion region 44 is formed between the filter medium fusion region 52 and a fused portion of the peripheral edge fusion region 50 with the inflow port 14. The proximal end 54 of the filter medium 18 is located in the non-fusion region 44. Specifically, the proximal end 54 of the filter medium 18 is located in the non-fusion region 44 beyond the filter medium fusion region 52.

Specifically, the inflow port 14 is integrally fused to the housing 12 in a state of being sandwiched between the peripheral edge fusion region 50 and the filter medium fusion region 52. Specifically, a portion of the inflow port 14 located in the non-fusion region 44 is fused only to the proximal end 54 of the filter medium 18. A portion of the inflow port 14 located in the non-fusion region 44 is not fused to the housing 12.

The non-fusion region 44 extends along the width direction of the housing 12. The width L1, which is a dimension of the non-fusion region 44 in the width direction, is larger than the width L2 (L1 > L2).

The window portion 46 is part of the housing 12. The window portion 46 is part of the first sheet portion 20 and the second sheet portion 22 constituting the housing 12. The window portion 46 is a window portion for allowing visual recognition of the non-fusion region 44 from the outside of the housing 12. The window portion 46 is provided in the housing 12 so as to face the non-fusion region 44. The window portion 46 is provided between the non-fusion region 44 and the peripheral edge fusion region 50 in the housing 12 so as to face the non-fusion region 44. Thus, in the front view of Fig. 14, a size of the window portion 46 is the same as that of the non-fusion region 44. Thus, a user can visually recognize the proximal end 54 of the filter medium 18 located in the non-fusion region 44 through the window portion 46. As illustrated in Fig. 15, the window portion 46 is provided in each of the first sheet portion 20 and the second sheet portion 22 so as to face the non-fusion region 44.

The window portion 46 is only required to be formed in at least one of the first sheet portion 20 or the second sheet portion 22.

In the medical filter 80 according to the third embodiment, when the filter medium 18 filters the liquid 49 such as blood, there is a possibility that the liquid 49 passes through a portion of the fusion region 32 where bonding force by fusion is low. Such a fusion abnormality is likely to occur in the fused portion between the filter medium fusion region 52 and the filter medium 18 and in the fused portion among the filter medium fusion region 52, the proximal portion 36 of the filter medium 18, and the inflow port 14. In other words, the bonding force by fusion is lower in the fused portion between the filter medium fusion region 52 and the filter medium 18 than in the peripheral edge fusion region 50. In addition, three members of the filter medium fusion region 52, the filter medium 18, and the inflow port 14 are fused, and thus, the bonding force by fusion tends to be lower than that in the peripheral edge fusion region 50.

Thus, if a fusion abnormality occurs, the liquid 49 passes through the filter medium fusion region 52 and flows into the non-fusion region 44. The user can confirm whether the liquid 49 flows into the non-fusion region 44 by visually recognizing the non-fusion region 44 through the window portion 46. In a case where the user confirms that the liquid 49 flows into the non-fusion region 44, the user can easily recognize that an abnormality has occurred in the medical filter 80.

Also in the third embodiment, the effects of the first embodiment (see Figs. 1 to 9) can be obtained. Further, the third embodiment further has the following effects.

As illustrated in Figs. 14 and 15, when the liquid 49 is introduced into the internal space 30 of the housing 12, in a case where the liquid 49 flows into the non-fusion region 44, a fusion abnormality occurs inside the medical filter 80. Thus, by visually recognizing inflow of the liquid 49 in the non-fusion region 44 from the outside of the housing 12 through the window portion 46, the user can detect an abnormality of the medical filter 80 at an early stage. In addition, early detection of an abnormality in the medical filter 80 makes it possible to reduce loss of the liquid 49 and suppress leakage of the liquid 49 to the outside of the medical filter 80.

The filter medium fusion region 52 is fused to the filter medium 18, and thus, the bonding force by fusion tends to be lower than that of the peripheral edge fusion region 50 which is a fused portion between the first sheet portion 20 and the second sheet portion 22. If a fusion abnormality occurs in the filter medium fusion region 52, the liquid 49 flows into the non-fusion region 44. This is effective for early detection of an abnormality of the medical filter 80.

The three members of the housing 12, the filter medium 18, and the inflow port 14 are fused, and thus, the bonding force by fusion tends to be lower in the fused portion among the filter medium fusion region 52, the proximal portion 36 of the filter medium 18, and the inflow port 14 than in the peripheral edge fusion region 50. If a fusion abnormality occurs in the fused portion, the liquid 49 flows into the non-fusion region 44 via the proximal end 54 of the filter medium 18. The proximal end 54 of the filter medium 18 is located in the non-fusion region 44 beyond the filter medium fusion region 52. In addition, the window portion 46 faces the non-fusion region 44. Thus, the user can visually recognize the proximal end 54 of the filter medium 18 located in the non-fusion region 44 through the window portion 46. In addition, the user can easily confirm whether the liquid 49 flows into the non-fusion region 44 through the window portion 46. This is effective for early detection of an abnormality of the medical filter 80.

The non-fusion region 44 extends along the width direction of the housing 12, and the width L1 of the non-fusion region 44 in the width direction is larger than the width L2 of the filter medium 18 in the width direction (L1 > L2). This can enlarge the window portion 46 facing the non-fusion region 44. This results in making it easier to view the non-fusion region 44 from the outside of the housing 12 through the window portion 46.

The width L3 of the outflow region 42 in the width direction is larger than the width L2 of the filter medium 18 in the width direction (L3 > L2). In addition, the filter medium fusion region 52 separates the non-fusion region 44 and the outflow region 42. If a fusion abnormality occurs in the filter medium fusion region 52, the liquid 49 flows into the non-fusion region 44 from the outflow region 42. This is more effective for early detection of an abnormality of the medical filter 80.

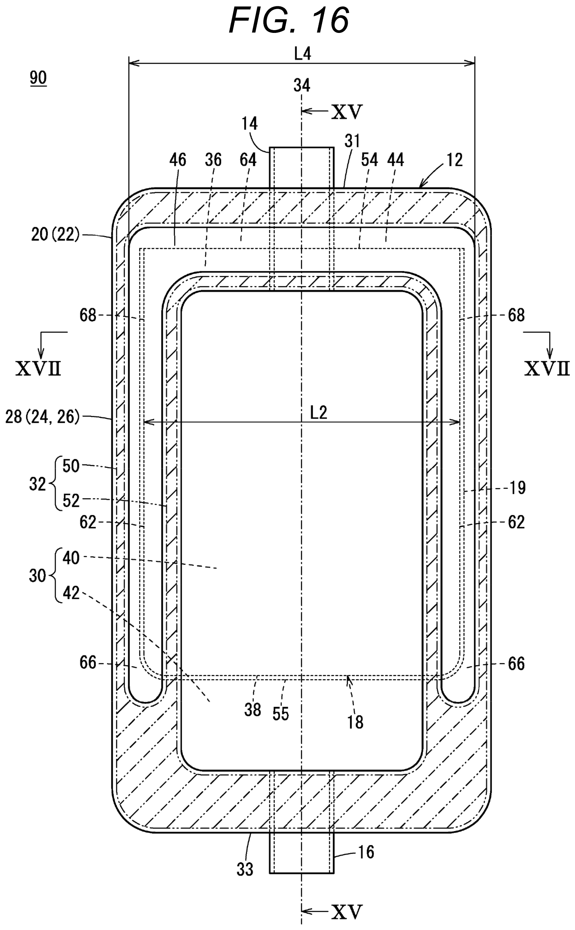

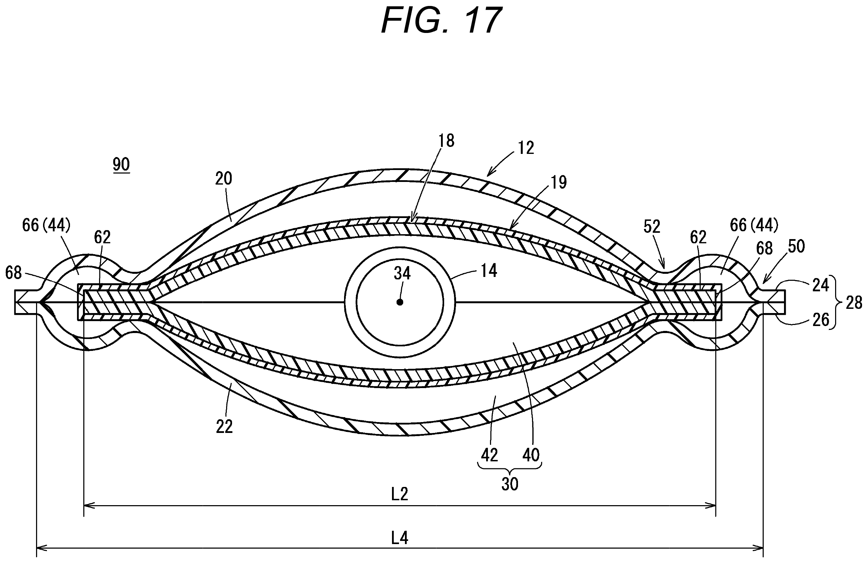

A medical filter 90 according to a fourth embodiment will be described with reference to Figs. 16 and 17.

In the medical filter 90 according to the fourth embodiment, the filter medium fusion region 52 is integrally fused to two side edge portions 62 between the proximal portion 36 and the distal portion 38 of the filter medium 18, the proximal portion 36 of the filter medium 18, and the inflow port 14. Thus, as illustrated in Fig. 16, the filter medium fusion region 52 is formed in a U shape. Note that the filter medium fusion region 52 may be integrally fused to at least one side edge portion 62 of the two side edge portions 62 of the filter medium 18, the proximal portion 36 of the filter medium 18, and the inflow port 14.

In the fourth embodiment, the non-fusion region 44 is formed in a U shape between the filter medium fusion region 52 and the peripheral edge fusion region 50. The non-fusion region 44 has a width direction portion 64 and two axial direction portions 66.

The width direction portion 64 is formed to face the proximal portion 36 of the filter medium 18. The width direction portion 64 extends along the width direction. The proximal end 54 of the filter medium 18 is located in the width direction portion 64. In other words, the proximal end 54 of the filter medium 18 is located in the width direction portion 64 beyond the filter medium fusion region 52.

Each of the two axial direction portions 66 is formed to face the side edge portion 62 of the filter medium 18. Each of the two axial direction portions 66 extends along the axial direction. Side edges 68 of the filter medium 18 are located in the axial direction portions 66. In other words, the side edge portions 62 of the filter medium 18 are located in the axial direction portions 66 beyond the filter medium fusion region 52.

The window portion 46 is provided in the housing 12 so as to face the non-fusion region 44. Thus, the window portion 46 is formed in a U shape. The user can visually recognize the proximal end 54 and the two side edge portions 62 of the filter medium 18 located in the non-fusion region 44 through the window portion 46. A width L4, which is a dimension of the width direction portion 64 in the width direction, is larger than the width L2 (L4 > L2). The axial direction portions 66 extend toward the outflow port 16 from the distal portion 38 of the filter medium 18.

Note that the width direction portion 64 and the two axial direction portions 66 may be divided. In addition, in a case where the filter medium fusion region 52 is integrally fused to one side edge portion 62 of the two side edge portions 62 of the filter medium 18, the proximal portion 36 of the filter medium 18, and the inflow port 14, the filter medium fusion region 52 may be formed in an L shape. In this case, the non-fusion region 44 and the window portion 46 are formed in an L shape so as to correspond to the filter medium fusion region 52.

Also in the fourth embodiment, the effects of the first embodiment (see Figs. 1 to 9) can be obtained. The fourth embodiment further has the following effects.

As illustrated in Figs. 16 and 17, three members of the housing 12, the filter medium 18, and the inflow port 14 are fused, and thus, the bonding force by fusion is likely to be lower in the fused portion among the filter medium fusion region 52, the proximal portion 36 of the filter medium 18, and the inflow port 14 than in the peripheral edge fusion region 50 which is the fused portion between the first sheet portion 20 and the second sheet portion 22. In addition, the bonding force by fusion tends to be lower in the fused portion between the filter medium fusion region 52 and the proximal portion 36 and the side edge portion 62 of the filter medium 18 than in the peripheral edge fusion region 50. If a fusion abnormality occurs in these fused portions, the liquid 49 flows into the non-fusion region 44 via the proximal end 54 and the side edge portions 62 of the filter medium 18. The proximal end 54 and the side edge portions 62 of the filter medium 18 are located in the non-fusion region 44 beyond the filter medium fusion region 52. In other words, the proximal end 54 and the side edge portions 62 of the filter medium 18 are not fused to the housing 12. The proximal end 54 and the side edge portions 62 of the filter medium 18 are free ends located in the non-fusion region 44. In addition, the window portion 46 faces the non-fusion region 44. Thus, the user can visually recognize the proximal end 54 and the side edge portions 62 of the filter medium 18 located in the non-fusion region 44 through the window portion 46. In addition, the user can easily confirm whether the liquid 49 flows into the non-fusion region 44 through the window portion 46. This is effective for early detection of an abnormality of the medical filter 90.

The width L4 of the width direction portion 64 in the width direction is larger than the width L2 of the filter medium 18 in the width direction (L4 > L2). This can enlarge a portion of the window portion 46 facing the width direction portion 64. This results in making it easier to view the width direction portion 64 from the outside of the housing 12 through the window portion 46.

If a fusion abnormality occurs in the filter medium fusion region 52, the liquid 49 flows into the non-fusion region 44 from the outflow region 42. This is more effective for early detection of an abnormality of the medical filter 90. Further, this can enlarge a portion of the window portion 46 facing the axial direction portions 66. This makes it easier to view the axial portion 66 from the outside of the housing 12 through the window portion 46.

Although the present disclosure has been described in detail, the present disclosure is not limited to the individual embodiments described above. Various additions, substitutions, modifications, partial deletions, and the like, may be made to these embodiments without departing from the gist of the present disclosure or without departing from the spirit of the present disclosure derived from the content described in the claims and equivalents thereof.

## Claims

1. A medical filter comprising:
a housing having an internal space;
an inflow port that is fixed to a peripheral edge portion of the housing and allows liquid to flow into the internal space from an outside of the housing;
a filter medium that is fixed to the housing and is disposed in the internal space;
an outflow port that is fixed to the peripheral edge portion and allows the liquid having passed through the filter medium to flow out to the outside; and
a reinforcing material that allows the liquid having passed through the filter medium to pass through and suppresses deformation of the filter medium due to a pressure of the liquid passing through the filter medium,
wherein
the filter medium separates the internal space into an inflow region into which the liquid flows through the inflow port and an outflow region from which the liquid having passed through the filter medium flows out to the outside through the outflow port, and
the reinforcing material
covers the filter medium in the outflow region, and
includes a parallel portion and a tapered portion connected to the parallel portion in a state in which the liquid passes through.

2. The medical filter according to claim 1, wherein
the reinforcing material has lower stretchability than the filter medium.

3. The medical filter according to claim 1 or 2, wherein
the filter medium is formed in a bag shape,
an inside of the filter medium is the inflow region,
the inflow port is inserted into the inflow region through a proximal portion of the filter medium, and
the reinforcing material is formed in a bag shape so as to cover an outer surface of the filter medium.

4. The medical filter according to claim 3, wherein
the proximal portion of the filter medium, the inflow port, and a proximal portion of the reinforcing material are integrally fixed to the peripheral edge portion of the housing.

5. The medical filter according to claim 3, wherein
the outer surface of the filter medium and an inner surface of the reinforcing material are in contact with each other.

6. The medical filter according to claim 5, wherein
the filter medium and the reinforcing material are integrally molded.

7. The medical filter according to claim 3, wherein
the parallel portion is parallel to an axial direction of the inflow port, and
the tapered portion is inclined with respect to the axial direction and connected to the parallel portion so as to surround the parallel portion.

8. The medical filter according to claim 1 or 2, wherein
the filter medium is a porous body that allows passage of the liquid, and
the reinforcing material is a porous body having a pore diameter larger than a pore diameter of the filter medium.

9. The medical filter according to claim 8, wherein
the reinforcing material is a nonwoven fabric.
